# EUROPEAN PATENT APPLICATION

(11) **EP 4 851 153 A1**
(43) Date of publication of application: **09.12.2026**
(21) Application number: 25748709.0
(22) Date of filing: 30.01.2025
(51) Int. Cl.: C07D 405/14, A61K 31/4545, A61K 45/00, A61P 21/00, A61P 25/00, A61P 25/14, A61P 25/16, A61P 25/28, A61P 43/00

(54) **CRYSTAL OF NITROGEN-CONTAINING SATURATED HETEROCYCLIC DERIVATIVE**

(30) Priority: 31.01.2024 JP 2024012411
(71) Applicant: Sumitomo Pharma Co., Ltd., Osaka-shi, Osaka 541-0045 (JP)
(72) Inventor: YAMADA, Shuya, Osaka-shi, Osaka 541-0045 (JP); NARAI, Shun, Osaka-shi, Osaka 554-0022 (JP)
(74) Representative: Maiwald GmbH
(86) International application number: PCT/JP2025/002925
(87) International publication number: WO 2025/164706

(57) **Abstract**

The present invention relates to a nitrogen-containing saturated heterocyclic derivative or a crystal thereof which has an action of suppressing or reducing the accumulation of abnormal protein aggregates in the brain, and a medicament comprising the derivative or a crystal thereof as an active ingredient, for treating or preventing central nervous system disease associated with abnormal protein aggregates in the brain.

## Description

### TECHNICAL FIELD

The present invention relates to a nitrogen-containing saturated heterocyclic derivative or a crystal thereof which has an action of suppressing or reducing the accumulation of abnormal protein aggregates in the brain, and a medicament comprising the derivative or a crystal thereof as an active ingredient, for treating or preventing central nervous system disease associated with abnormal protein aggregates in the brain.

### BACKGROUND OF THE INVENTION

Neurodegenerative diseases which mainly include Alzheimer disease, Parkinson disease, Huntington disease, and amyotrophic lateral sclerosis are thought to be caused by the formation of abnormally-aggregated proteins in a patient's brain, the aggregates exhibiting neurotoxicity, and thereby the onset and progression of the diseases.

Constituent proteins of the aggregates differ depending on diseases, and α-synuclein has been reported as a major constituent of aggregates that cause Parkinson disease. It has been reported that abnormally-aggregated α-synuclein exhibits neurotoxicity, and that aggregated α-synuclein propagates between cells.

As an agent for treating Parkinson disease, the administration of levodopa which is a dopamine precursor is available as symptomatic therapy, but no fundamental treatment has been established at present. In recent years, some developments of disease-modifying drugs for Parkinson disease have been vigorously pursued, but there are currently no reports of agents undergoing clinical trials that strongly suppress or reduce the accumulation of α-synuclein aggregates.

It is considered that α-synuclein aggregates are the causative background of Lewy body diseases including Parkinson disease (dementia with Lewy body, multiple system atrophy, Gaucher disease, infantile neuroaxonal dystrophy, etc.). Accordingly, drugs that suppress or reduce the accumulation of α-synuclein aggregates are expected to exhibit therapeutic effects on these diseases.

Until now, no *in vitro* evaluation system that reproduces endogenous α-synuclein aggregates in neurons has been reported, and many evaluation systems for α-synuclein pathology are indicated by the amount of phosphorylated α-synuclein increased due to the addition of *in vitro-*synthesized α-synuclein oligomers. After all, it has been impossible to evaluate an action of suppressing the accumulation of α-synuclein aggregates or reducing accumulated α-synuclein aggregates.

Until now, NPT200-11 (Neuropore) and Anle138b (MODAG) have been reported as drugs that can inhibit the formation of α-synuclein aggregates. However, these drugs had been evaluated about the action of inhibiting the ability to form aggregates when artificially aggregating α-synuclein *in vitro* (Patent literatures 1 and 2).

It has also been reported that (4aR,8aS)-hexahydro-2H-pyrido[4,3-b][1,4]oxazin-3(4H)-one derivatives such as (4aR,8aS)-6-{4-[5-(trifluoromethyl)pyridin-3-yl]piperidine-1-carbonyl}hexahydro-2H-pyrido[4,3-b][1,4]oxazin-3(4H)-one and (4aR,8aS)-6-[4-(5-ethylpyridin-3-yl)piperidine-1-carbonyl]hexahydro-2H-pyrido[4,3-b][1,4]oxazin-3(4H)-one have an action of inhibiting monoacylglycerol lipase (MAGL), which are effective for neuroinflammation, neurodegenerative disease, etc. (Patent literature 3). In addition, It has also been reported that (azetidin-1-yl)(phenyl)methanone derivatives such as 2-chloro-3-{3-[6-(trifluoromethyl)pyridin-3-yl]azetidine-1-carbonyl}-4-[(1,1,1-trifluoropropan-2-yl)oxy]benzonitrile and 2-methoxy-3-{3-[6-(trifluoromethyl)pyridin-3-yl]azetidine-1-carbonyl}-4-[(1,1,1-trifluoropropan-2-yl)oxy]benzonitrile have an action of inhibiting glycine transporter 1 (GlyT1), which are effective for neurodegenerative disease, etc. (Patent literature 4).

However, all of these compounds are structurally distinct from the nitrogen-containing saturated heterocyclic derivative of the present invention. These documents neither disclose nor provide any teaching or suggestion about the nitrogen-containing saturated heterocyclic derivative of the present invention or a crystal thereof. In addition, they provide no teaching or suggestion regarding the action of suppressing or reducing the accumulation of abnormal protein aggregates in the brain.

### PRIOR ART

### (Patent Reference)

[Patent literature 1] WO 2011/084642
[Patent literature 2] WO 2010/000372
[Patent literature 3] WO 2019/180185
[Patent literature 4] WO 2016/073420

### SUMMARY OF THE INVENTION

### (Technical Problem)

An object of the present invention is to provide a compound or a crystal thereof for use in preventing or treating central nervous system disease, which is characterized by an action of suppressing or reducing the accumulation of abnormal protein aggregates in the brain.

### (Solution to Problem)

The present inventors have conducted extensive studies to achieve the above object, and then have found that a nitrogen-containing saturated heterocyclic derivative, (3-methyloxetan-3-yl){4-[6-(trifluoromethyl)pyridin-3-yl]piperidin-1-yl}methanone (referred to as "the present compound" as appropriate) has an action of suppressing or reducing the accumulation of abnormal protein aggregates in the brain, and have also found a crystal of (3-methyloxetan-3-yl){4-[6-(trifluoromethyl)pyridin-3-yl]piperidin-1-yl}methanone in stable crystalline form I. Based upon the findings, the present invention has been achieved. The present invention is as described below.

### [Item 1]

A crystal of (3-methyloxetan-3-yl){4-[6-(trifluoromethyl)pyridin-3-yl]piperidin-1-yl}methanone in crystalline form I, which is characterized by X-ray powder diffraction pattern having diffraction angle (2θ°) peaks of 17.8° ± 0.2° and 19.6° ± 0.2°.

### [Item 2]

A crystal of (3-methyloxetan-3-yl){4-[6-(trifluoromethyl)pyridin-3-yl]piperidin-1-yl}methanone in crystalline form I, which is characterized by X-ray powder diffraction pattern having four or more diffraction angle (2θ°) peaks selected from 16.6° ± 0.2°, 17.2° ± 0.2°, 17.8° ± 0.2°, 18.3° ± 0.2°, 19.6° ± 0.2°, 19.9° ± 0.2°, 22.0° ± 0.2°, 22.8° ± 0.2°, 23.9° ± 0.2°, and 25.2° ± 0.2°.

### [Item 3]

A medicament comprising the crystal of (3-methyloxetan-3-yl){4-[6-(trifluoromethyl)pyridin-3-yl]piperidin-1-yl}methanone in crystalline form I according to Item 1 or 2 as an active ingredient.

### [Item 4]

A medicament for treating or preventing central nervous system diseases associated with abnormal protein aggregates in the brain, comprising the crystal of (3-methyloxetan-3-yl){4-[6-(trifluoromethyl)pyridin-3-yl]piperidin-1-yl}methanone in crystalline form I according to Item 1 or 2 as an active ingredient.

### [Item 5]

The medicament of Item 4, wherein the central nervous system diseases associated with abnormal protein aggregates in the brain are central nervous system diseases related to tau, α-synuclein, TDP-43, or polyglutamine.

### [Item 6]

The medicament of Item 4, wherein the central nervous system diseases associated with abnormal protein aggregates in the brain are Alzheimer disease, frontotemporal lobar degeneration, Parkinson disease, Lewy body dementia, multiple system atrophy, Gaucher disease, infantile neuroaxonal dystrophy, amyotrophic lateral sclerosis, Huntington disease, or spinocerebellar ataxia.

### [Item 7]

The medicament of Item 4, wherein the central nervous system diseases associated with abnormal protein aggregates in the brain are central nervous system diseases related to α-synuclein.

### [Item 8]

The medicament of Item 4, wherein the central nervous system diseases associated with abnormal protein aggregates in the brain are Parkinson disease, Lewy body dementia, multiple system atrophy, Gaucher disease, or infantile neuroaxonal dystrophy.

### [Item 9]

A method for treating and/or preventing central nervous system diseases associated with abnormal protein aggregates in the brain, comprising administering a therapeutically effective amount of the crystal of (3-methyloxetan-3-yl){4-[6-(trifluoromethyl)pyridin-3-yl]piperidin-1-yl}methanone in crystalline form I according to Item 1 or 2 to a patient in need thereof.

### [Item 10]

Use of the crystal of (3-methyloxetan-3-yl){4-[6-(trifluoromethyl)pyridin-3-yl]piperidin-1-yl}methanone in crystalline form I according to Item 1 or 2, in the manufacture of a medicament for treating and/or preventing central nervous system diseases associated with abnormal protein aggregates in the brain.

### [Item 11]

The crystal of (3-methyloxetan-3-yl){4-[6-(trifluoromethyl)pyridin-3-yl]piperidin-1-yl}methanone in crystalline form I according to Item 1 or 2 for use in treating and/or preventing central nervous system diseases associated with abnormal protein aggregates in the brain.

### [Item 12]

A medicament for treating or preventing central nervous system diseases associated with abnormal protein aggregates in the brain, comprising a combination of the crystal of (3-methyloxetan-3-yl){4-[6-(trifluoromethyl)pyridin-3-yl]piperidin-1-yl}methanone in crystalline form I according to Item 1 or 2, and at least one drug selected from the group consisting of L-dopa, dopamine agonist, MAO-B inhibitor, catechol-O-methyltransferase (COMT) inhibitor, αSyn antibody, and pharmaceutically acceptable salts thereof.

### [Item 13]

A medicament for treating or preventing central nervous system diseases associated with abnormal protein aggregates in the brain, comprising the crystal of (3-methyloxetan-3-yl){4-[6-(trifluoromethyl)pyridin-3-yl]piperidin-1-yl}methanone in crystalline form I according to Item 1 or 2, which is used in combination with at least one drug selected from the group consisting of L-dopa, dopamine agonist, MAO-B inhibitor, catechol-O-methyltransferase (COMT) inhibitor, αSyn antibody, and pharmaceutically acceptable salts thereof.

### (Effect of the Invention)

The present invention provides (3-methyloxetan-3-yl){4-[6-(trifluoromethyl)pyridin-3-yl]piperidin-1-yl}methanone or a crystal thereof. The compound or a crystal thereof has an action of suppressing or reducing the accumulation of abnormal protein aggregates in the brain, thereby making it useful as a medicament for treating or preventing central nervous system disease associated with abnormal protein aggregates in the brain, in particular, neurodegenerative disease related to α-synuclein such as Parkinson disease, Lewy body dementia, multiple system atrophy, Gaucher disease, and infantile neuroaxonal dystrophy. In addition, a crystal of (3-methyloxetan-3-yl){4-[6-(trifluoromethyl)pyridin-3-yl]piperidin-1-yl}methanone in crystalline form I has a high melting point and excellent thermal stability and hygroscopic stability, thereby making it useful as a pharmaceutical product.

### BRIEF DESCRIPTION OF THE DRAWINGS

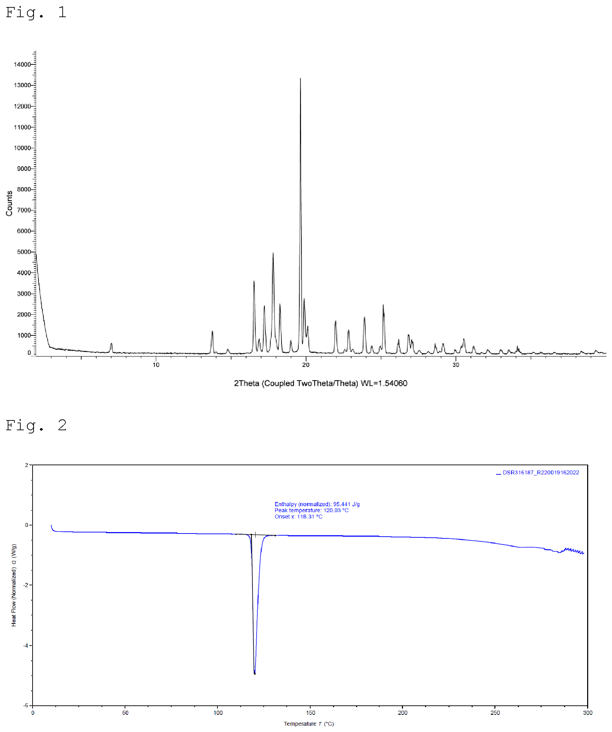

Fig. 1 shows the X-ray powder diffraction pattern of the compound (crystalline form I) prepared in Example 2, which was conducted in Example 3. The x-axis represents the 2θ value, and the y-axis represents the intensity.
Fig. 2 shows the differential scanning calorimetry (DSC) of the compound (crystalline form I) prepared in Example 2, which was conducted in Example 4. The x-axis represents temperature (°C), and the y-axis represents heat flow (W/g).

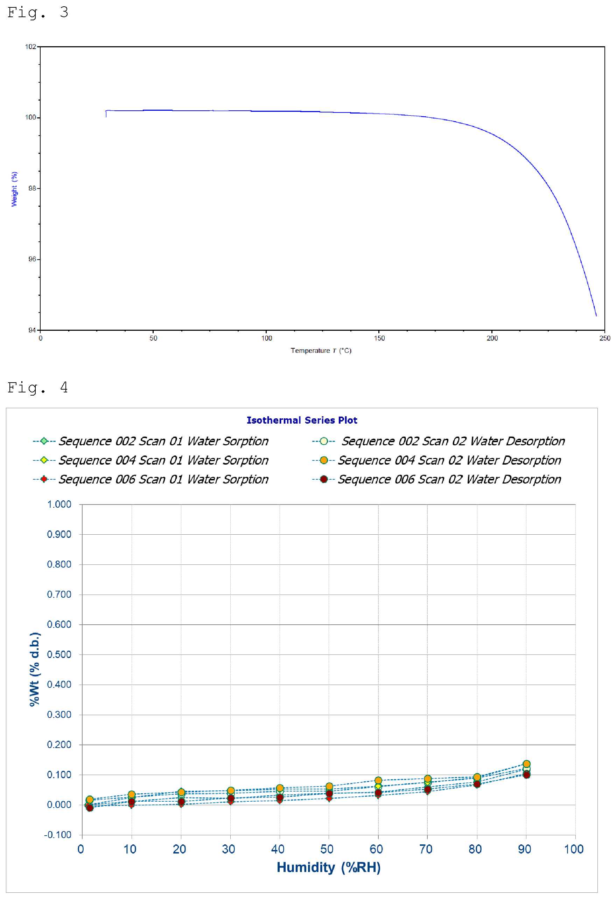

Fig. 3 shows the thermogravimetric analysis (TGA) of the compound (crystalline form I) prepared in Example 2, which was conducted in Example 5. The x-axis represents temperature (°C), and the y-axis represents weight change (%).
Fig. 4 shows the dynamic vapor sorption (DVS) of the compound (crystalline form I) prepared in Example 2, which was conducted in Example 6. The x-axis represents relative humidity (%), and the y-axis represents mass change (%).

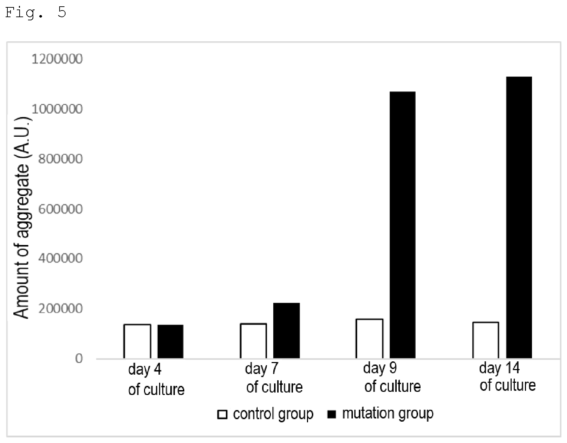

Fig. 5 shows the difference in the amount of aggregates in the neurospheroid derived from healthy human iPS cells and in the neurospheroid derived from PLA2G6 mutant iPS cells. The vertical axis indicates the amount of aggregate in neurospheroid, and the horizontal axis indicates the number of culture days. The white bars indicate the aggregate amount of in the neurospheroid derived from healthy human iPS cells, and the black bars indicate the aggregate amount of in the neurospheroid derived from PLA2G6 mutant iPS cells.

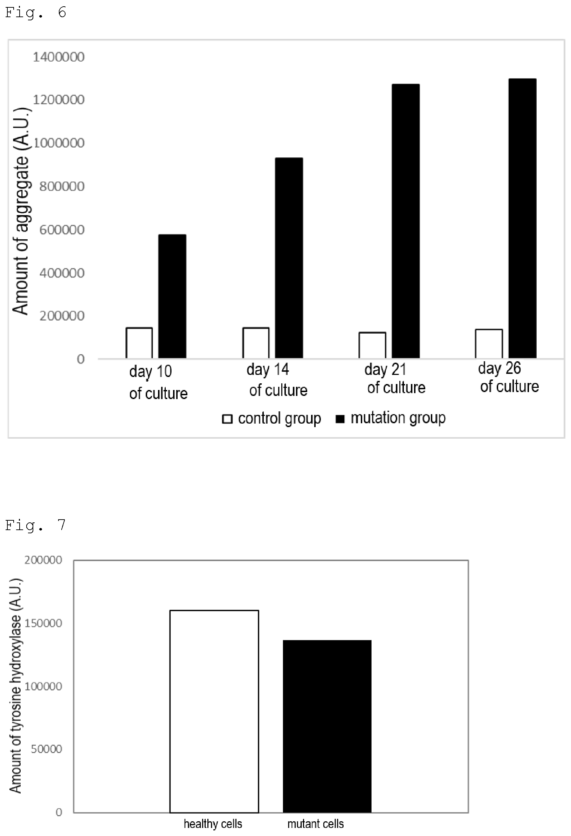

Fig. 6 shows the difference in the amount of aggregates in the dopamine neurospheroid derived from healthy human iPS cells and in the dopamine neurospheroid derived from PLA2G6 mutant iPS cells. The vertical axis indicates the amount of aggregate in dopamine neurospheroid, and the horizontal axis indicates the number of culture days. The white bars indicate the aggregate amount of in the neurospheroid derived from healthy human iPS cells, and the black bars indicate the aggregate amount of in the neurospheroid derived from PLA2G6 mutant iPS cells.
Fig. 7 shows the difference in the amount of tyrosine hydroxylase in the dopamine neurospheroid derived from healthy human iPS cells and in the dopamine neurospheroid derived from PLA2G6 mutant iPS cells on day 26 of culture. The vertical axis indicates the amount of tyrosine hydroxylase in dopamine neurospheroid. The white bar indicates the amount of tyrosine hydroxylase in the neurospheroid derived from healthy human iPS cells, and the black bar indicates the amount of tyrosine hydroxylase in the neurospheroid derived from PLA2G6 mutant iPS cells.

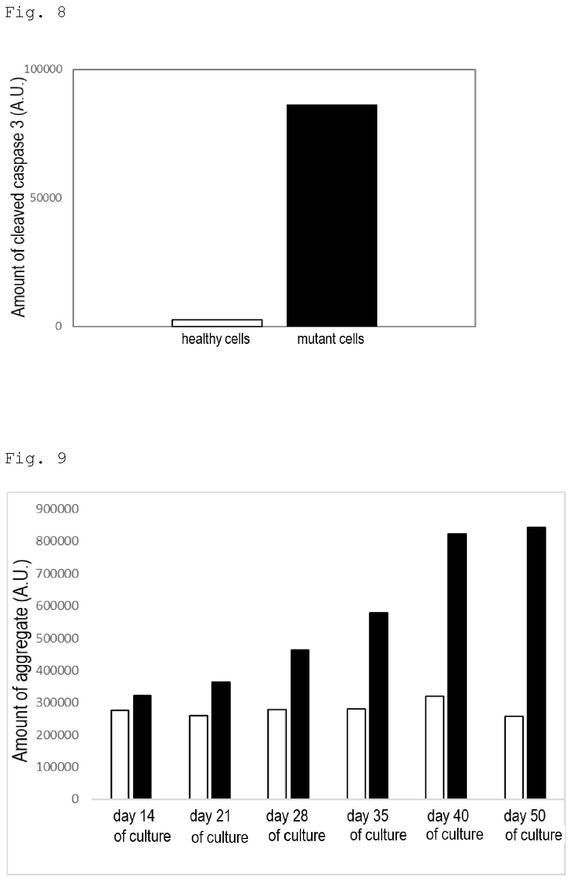

Fig. 8 shows the difference in the amount of cleaved caspase 3 in the dopamine neurospheroid derived from healthy human iPS cells and in the dopamine neurospheroid derived from PLA2G6 mutant iPS cells on day 40 of culture. The vertical axis indicates the amount of cleaved caspase 3 in dopamine neurospheroid. The white bar indicates the amount of cleaved caspase 3 in the neurospheroid derived from healthy human iPS cells, and the black bar indicates the amount of cleaved caspase 3 in the neurospheroid derived from PLA2G6 mutant iPS cells.
Fig. 9 shows the difference in the amount of aggregates in the dopamine neurospheroid derived from healthy human iPS cells and in the dopamine neurospheroid derived from GBA1 gene homozygous mutant iPS cells. The vertical axis indicates the amount of aggregate in dopamine neurospheroid, and the horizontal axis indicates the number of culture days. The white bars indicate the aggregate amount of in the neurospheroid derived from healthy human iPS cells, and the black bars indicate the aggregate amount of in the neurospheroid derived from GBA1 gene homozygous mutant iPS cells.

### DETAILED DESCRIPTION OF THE PREFERRED EMBODIMENTS

Hereinafter, the present invention is explained in detail.

The compound of the present invention, (3-methyloxetan-3-yl){4-[6-(trifluoromethyl)pyridin-3-yl]piperidin-1-yl}methanone, has the following structure.

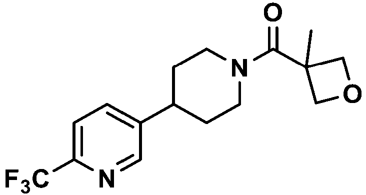

(3-Methyloxetan-3-yl){4-[6-(trifluoromethyl)pyridin-3-yl]piperidin-1-yl}methanone forms a crystal. It is generally known that crystalline substances can be analyzed using conventional techniques such as X-ray powder diffraction (hereinafter, "XRPD") analysis, differential scanning calorimetry (hereinafter, "DSC"), thermogravimetry (hereinafter, "TGA"), dynamic vapor sorption (hereinafter, "DVS"), diffuse reflection infrared Fourier transform (DRIFT) spectroscopy, near infrared (NIR) spectroscopy, liquid-phase and/or solid-phase nuclear magnetic resonance spectroscopy. The crystal of the present compound in crystalline form I is an anhydrate, but may contain a small amount of water. The water content of a crystalline substance can be measured by Karl-Fischer analysis.

For example, although one crystal form (crystalline form I) is identified herein as a crystal of (3-methyloxetan-3-yl){4-[6-(trifluoromethyl)pyridin-3-yl]piperidin-1-yl}methanone, the crystal form of the present invention is not limited to this crystal form.

An embodiment of the present invention includes (3-methyloxetan-3-yl){4-[6-(trifluoromethyl)pyridin-3-yl]piperidin-1-yl}methanone in crystalline form I.

A further embodiment of the present invention includes (3-methyloxetan-3-yl){4-[6-(trifluoromethyl)pyridin-3-yl]piperidin-1-yl}methanone that is in crystalline form I, which is characterized by X-ray powder diffraction pattern having diffraction angle (2θ°) peak of at least 19.6° ± 0.2°.

A further embodiment of the present invention includes (3-methyloxetan-3-yl){4-[6-(trifluoromethyl)pyridin-3-yl]piperidin-1-yl}methanone that is in crystalline form I, which is characterized by X-ray powder diffraction pattern having diffraction angle (2θ°) peak of at least 17.8° ± 0.2°.

A further embodiment of the present invention includes (3-methyloxetan-3-yl){4-[6-(trifluoromethyl)pyridin-3-yl]piperidin-1-yl}methanone that is in crystalline form I, which is characterized by X-ray powder diffraction pattern having diffraction angle (2θ°) peaks of at least 17.8° ± 0.2° and 19.6° ± 0.2°.

A further embodiment of the present invention includes (3-methyloxetan-3-yl){4-[6-(trifluoromethyl)pyridin-3-yl]piperidin-1-yl}methanone that is in crystalline form I, which is characterized by X-ray powder diffraction pattern having diffraction angle (2θ°) peaks of 16.6° ± 0.2°, 17.2° ± 0.2°, 17.8° ± 0.2°, 18.3° ± 0.2°, 19.6° ± 0.2°, 19.9° ± 0.2°, 22.0° ± 0.2°, 22.8° ± 0.2°, 23.9° ± 0.2°, and 25.2° ± 0.2°. The crystal in the present invention is characterized by having four or five peaks selected from these ten peaks.

A further embodiment of the present invention includes (3-methyloxetan-3-yl){4-[6-(trifluoromethyl)pyridin-3-yl]piperidin-1-yl}methanone that is in crystalline form I, which has X-ray powder diffraction pattern substantially the same as the X-ray powder diffraction pattern shown in Figure 1.

A further embodiment of the present invention includes (3-methyloxetan-3-yl){4-[6-(trifluoromethyl)pyridin-3-yl]piperidin-1-yl}methanone that is in crystalline form I, which has an endothermic peak associated with melting having an extrapolated onset temperature (Tim) of 120.0°C ± 5°C in differential scanning calorimetry (DSC) analysis.

A further embodiment of the present invention includes (3-methyloxetan-3-yl){4-[6-(trifluoromethyl)pyridin-3-yl]piperidin-1-yl}methanone that is in crystalline form I, which has a DSC curve substantially the same as the DSC curve shown in Figure 2.

A further embodiment of the present invention includes (3-methyloxetan-3-yl){4-[6-(trifluoromethyl)pyridin-3-yl]piperidin-1-yl}methanone that is in crystalline form I, which has a TGA curve substantially the same as the TGA curve shown in Figure 3.

A further embodiment of the present invention includes (3-methyloxetan-3-yl){4-[6-(trifluoromethyl)pyridin-3-yl]piperidin-1-yl}methanone that is in crystalline form I, which has a DVS graph substantially the same as the DVS graph shown in Figure 4.

The crystal of (3-methyloxetan-3-yl){4-[6-(trifluoromethyl)pyridin-3-yl]piperidin-1-yl}methanone in crystalline form I can be prepared by adding hexane to an ethyl acetate solution of Example 1 and allowing the mixture to stand.

The crystal of (3-methyloxetan-3-yl){4-[6-(trifluoromethyl)pyridin-3-yl]piperidin-1-yl}methanone in crystalline form I can be prepared by crystallization or drying from one or a combination of two or more (for example, 6 to 7) various solvents (for example, water, methanol, ethanol, isopropanol, chloroform, acetonitrile, 1,2-dimethoxyethane, tetrahydrofuran, 1,4-dioxane, diisopropyl ether, cyclopropyl methyl ether, ethyl acetate, isopropyl acetate, isobutyl acetate, acetone, chlorobenzene, toluene, xylene, water/methanol mixture (1:1), water/isopropanol mixture (1:1), water/acetone mixture (1:1), water/acetonitrile mixture (1:1), water/tetrahydrofuran mixture (1:1), water/1,4-dioxane mixture (1:1), water/ethyl acetate mixture (1:1), heptane/acetone mixture (1:1), heptane/acetonitrile mixture (1:1), heptane/tetrahydrofuran mixture (1:1), heptane/toluene mixture (1:1), etc.) under conditions ranging from room temperature to refrigerated conditions, or from rapid cooling to freezing conditions. Alternatively, the crystal can be prepared by shaking suspensions in the respective solvents described in Example 9 (such as water, ethanol, isopropanol, chloroform, acetonitrile, tetrahydrofuran, ethyl acetate, acetone, chlorobenzene, toluene, water/ethanol mixture (1:1), water/isopropanol mixture (1:1), water/tetrahydrofuran mixture (1:1), water/acetone mixture (1:1), water/acetonitrile mixture (1:1), etc.) at room temperature to about 50°C for up to 14 days.

Since the 2θ values of the X-ray powder diffraction pattern may vary slightly depending on the instrument or sample, the numerical values described herein are not absolute (*see*, Jenkins, R & Snyder, R.L. 'Introduction to X-Ray Powder Diffractometry' John Wiley & Sons 1996; Bunn, C.W. (1948), Chemical Crystallography, Clarendon Press, London; Klug, H. P. & Alexander, L. E. (1974), X-Ray Diffraction Procedures). In general, the measurement error of diffraction angles in an X-ray powder diffraction spectrum is, for example, about ± 0.2° in 2θ, and such a degree of measurement error should be taken into consideration when analyzing X-ray powder diffraction data. Furthermore, the intensity may vary depending on the experimental conditions and sample preparation (preferred orientation). In the present specification, the values are shown as measured using copper radiation (Cu Kα1, λ = 1.5406 Å, Kα2, λ = 1.5444 Å).

It is also known that an X-ray powder diffraction pattern having one or more measurement errors may be obtained depending on the measurement conditions (for example, the instrument or apparatus used). For example, crystal particles having a size greater than 30 microns or having non-uniform aspect ratios may affect the relative intensities of the peaks. In addition, the positions of the reflections may be affected by the exact height at which the sample is placed in the diffractometer and by the zero calibration of the diffractometer. The planarity of the sample surface may also have some influence. Accordingly, unless otherwise specified, the crystal forms of the present invention described above are not limited to crystals that provide the X-ray powder diffraction pattern shown in Figure 1, and any crystals that provide an X-ray powder diffraction pattern substantially the same as the X-ray powder diffraction pattern shown in these figures are included within the scope of the present invention.

Further, in differential scanning calorimetry (DSC), a tolerance of ± 5°C is allowed for the extrapolated onset temperature (Tim), endothermic peak temperature (Tpm), and the like. The extrapolated onset temperature (Tim) in differential scanning calorimetry (DSC) refers to the temperature at the point where the extrapolated line of the rising portion of the endothermic peak curve intersects with the extrapolated baseline, while the endothermic peak temperature (Tpm) refers to the temperature at the top of the endothermic peak.

Processes to prepare (3-methyloxetan-3-yl){4-[6-(trifluoromethyl)pyridin-3-yl]piperidin-1-yl}methanone or a salt thereof in the present invention are explained below with examples, but the present invention should not be limited thereto.

### Preparation Process

(3-Methyloxetan-3-yl){4-[6-(trifluoromethyl)pyridin-3-yl]piperidin-1-yl}methanone may be synthesized according to each Preparation Process shown below or its combination with a known synthetic process.

Each compound in the following schemes may exist as a salt thereof, wherein the salt includes a salt in conventional organic synthesis. The following schemes are disclosed as just examples, thus it is also possible to optionally prepare the present compound by a different process based on the knowledge of a skilled person in synthetic organic chemistry field.

In each Preparation Process described below, protecting groups can be used as necessary, even if the use of protecting groups is not explicitly stated. And, the protecting groups can be deprotected after a reaction has been completed or a series of reactions have been carried out to obtain the desired compound.

The introduction and removal of protecting groups can be carried out by a method commonly used in organic synthetic chemistry, for example, methods described in T. W. Greene and P. G. M. Wuts, "Protective Groups in Organic Synthesis", 3rd Ed., John Wiley and Sons, inc., New York (1999), or a method analogous thereto.

Examples of amino-protecting groups include, for example, tert-butoxycarbonyl, benzyloxycarbonyl, p-toluenesulfonyl, o-nitrobenzenesulfonyl, 4-methoxybenzyl, 2,4-dimethoxybenzyl, and the like.

### Preparation process 1

The compound of formula (1) can be prepared, for example, by the following preparation process. 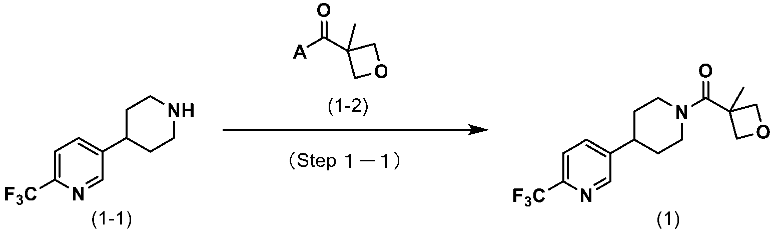 Wherein A is halogen or OH.

### (Step 1-1: Preparation step of Compound (1))

Compound (1) can be prepared by reacting Compound (1-1) and Compound (1-2) in a suitable inert solvent in the presence of any condensing agent and/or any base, or in the absence of them. As Compound (1-1), a commercial compound may be used, or a product prepared by a known method (for example, WO 2014/192868) may be used. Alternatively, the products prepared in the manner described in Preparation process 2 or 3 shown below may be used as Compound (1-1). As Compound (1-2), a commercial compound may be used, or a product prepared by a known method (for example, WO 2016/004272) may be used. The base used in the present step may be suitably selected from the bases exemplified below, which includes, for example, sodium hydride, triethylamine, diisopropylethylamine, and sodium carbonate. The condensing agent used in the present step may be selected from various condensing agents which are used generally in organic synthetic reaction, which includes, for example, 1-ethyl-3-(3-dimethylaminopropyl)carbodiimide hydrochloride, 1-hydroxybenzotriazole, 1-[bis(dimethylamino)methylene]-1H-1,2,3-triazolo[4,5-b]pyridinium 3-oxide hexafluorophosphate, and 2,4,6-tripropyl-1,3,5,2,4,6-trioxatriphosphorinane-2,4,6-trioxide. The solvent used in the present step may be suitably selected from the solvents exemplified below, which includes, for example, DMF, THF, dichloromethane, chloroform, and ethyl acetate. The reaction time in the present step is generally 5 minutes to 72 hours, preferably 30 minutes to 24 hours. The reaction temperature in the present step is generally -78°C to 200°C, preferably -78°C to 80°C.

### Preparation process 2

The compound of formula (1-1) can be prepared, for example, by the following preparation process. 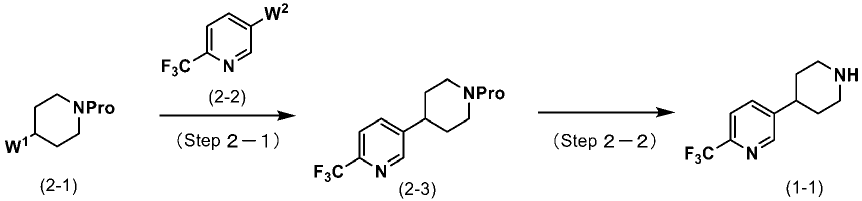 Wherein W¹ and W² are halogen; and Pro is an amino-protecting group.

### (Step 2-1: Preparation step of Compound (2-3))

Compound (2-3) can be prepared by reacting Compound (2-1) and Compound (2-2) in a suitable inert solvent in the presence of zinc and a palladium catalyst. As Compound (2-1), a commercial compound may be used, or a product prepared by a known method (for example, WO 2008/147831) may be used. As Compound (2-2), a commercial compound may be used, or a product prepared by a known method (for example, Bioorganic & Medicinal Chemistry Letters (2006), 16(17), 4528-4532) may be used. The halogen in W¹ and W² includes, for example, chlorine, bromine, and iodine. The amino-protecting group, Pro, includes tert-butoxycarbonyl group, and benzyloxycarbonyl group. The palladium catalyst used herein includes various palladium catalysts commonly used in conventional methods, for example, tetrakis(triphenylphosphine)palladium(0). The solvent used in the present step may be suitably selected from the solvents exemplified below, which includes, for example, DMF and dimethylacetamide. The reaction time in the present step is generally 5 minutes to 48 hours, preferably 1 hour to 24 hours. The reaction temperature in the present step is generally 0°C to 100°C, preferably 0°C to 80°C.

### (Step 2-2: Preparation step of Compound (1-1))

Compound (1-1) can be prepared by deprotecting the amino-protecting group Pro of Compound (2-3) by a known method (for example, Protective Group in Organic Synthesis, 3rd ed. (Theodora W. Green, Peter G. M. Wuts, John Wiley & Sons Inc, 1999)).

### Preparation process 3

The compound of formula (1-1) can be also prepared, for example, by the following preparation process. 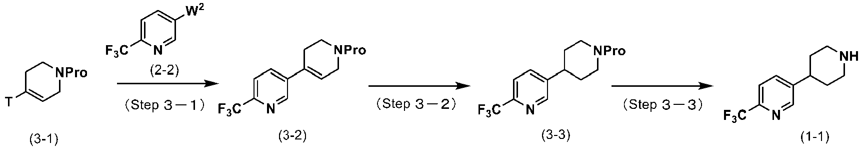 Wherein W² is halogen; Pro is an amino-protecting group; T is a boronic acid or boronate ester.

### (Step 3-1: Preparation step of Compound (3-2))

Compound (3-2) can be prepared by reacting Compound (3-1) and Compound (2-2) in a suitable inert solvent in the presence of a palladium catalyst. The present step can be carried out in the presence of a base and/or a phosphorus ligand, if necessary. As Compound (3-1), a commercial compound may be used, or a product prepared by a known method (for example, WO 2019/163865) may be used. The amino-protecting group, Pro, includes tert-butoxycarbonyl group and benzyloxycarbonyl group. The palladium catalyst used herein includes various palladium catalysts commonly used in conventional methods, for example, tetrakis(triphenylphosphine)palladium(0). The base used in the present step may be suitably selected from the bases exemplified below, which includes, for example, potassium carbonate and cesium carbonate. The phosphorus ligand used herein includes various phosphorus ligands commonly used in organic synthetic reactions, for example, triphenylphosphine and bis(diphenylphosphino)methane. The solvent used in the present step may be suitably selected from the solvents exemplified below, which includes, for example, 1,4-dioxane, tetrahydrofuran, water, and a mixture thereof. The reaction temperature in the present step is generally 0°C to 200°C, preferably 20°C to 150°C, and the reaction may also be carried out under microwave irradiation, if necessary. The reaction time varies depending on conditions such as the reaction temperature, the palladium catalyst used, the starting materials, and the solvent, but is usually from 5 minutes to 72 hours, preferably from 1 hour to 24 hours.

### (Step 3-2: Preparation step of Compound (3-3))

Compound (3-3) can be prepared by reacting Compound (3-2) under hydrogen atmosphere in a suitable inert solvent in the presence of a catalyst. The solvent used in the present step may be suitably selected from the solvents exemplified below, which includes, for example, methanol, ethanol, chloroform, and a mixture thereof. The catalyst used herein includes various catalysts commonly used in catalytic hydrogenation reactions, for example, palladium carbon and palladium hydroxide. The reaction time in the present step is generally 5 minutes to 48 hours, preferably 1 hour to 24 hours. The reaction temperature in the present step is generally 0°C to 100°C, preferably 0°C to 40°C.

### (Step 3-3: Preparation step of Compound (1-1))

Compound (1-1) can be prepared from Compound (3-3) according to the process shown in Step 2-2.

Among the starting materials and the intermediates in each preparation process mentioned above, the compounds that are not described in each process are commercially available or can be prepared by a skilled person with a commercially available material in a known manner or a similar manner thereto.

The base used in each step of the above processes should be suitably selected based on the reaction, the starting compound, etc., which includes, for example, alkaline bicarbonates such as sodium bicarbonate, and potassium bicarbonate; alkaline carbonate such as sodium carbonate, and potassium carbonate; metallic hydrides such as sodium hydride, and potassium hydride; alkaline metal hydroxides such as sodium hydroxide, and potassium hydroxide; alkaline metal alkoxides such as sodium methoxide, and sodium t-butoxide; organic metal bases such as butyllithium, and lithium diisopropylamide; and organic bases such as triethylamine, diisopropylethylamine, pyridine, 4-dimethylaminopyridine (DMAP), and 1,8-diazabicyclo[5.4.0]undec-7-ene (DBU).

The solvent used in each step of the above processes should be suitably selected based on the reaction, the starting compound, etc., which includes, for example, alcohol solvents such as methanol, ethanol, and isopropanol; ketone solvents such as acetone and methylketone; halogenated hydrocarbon solvents such as methylene chloride and chloroform; ether solvents such as tetrahydrofuran (THF) and dioxane; aromatic hydrocarbon solvents such as toluene and benzene; aliphatic hydrocarbon solvents such as hexane and heptane; ester solvents such as ethyl acetate and propyl acetate; amide solvents such as N,N-dimethylformamide (DMF) and N-methyl-2-pyrrolidone (NMP); sulfoxide solvents such as dimethylsulfoxide (DMSO); and nitrile solvents such as acetonitrile. The solvent used herein may be one of these solvents or a mixture of two or more solvents selected from these solvents. And, if possible in the reaction, an organic base such as diazabicycloundecene (DBU) may be used as a solvent used herein.

The suitable salts of starting materials and intermediates and the acceptable salts of drug substances are conventional non-toxic salts. The suitable salt includes, for example, acid addition salts such as organic acid salts (including acetate, trifluoroacetate, maleate, fumarate, citrate, tartrate, methanesulfonate, benzenesulfonate, formate, and para-toluenesulfonate) and inorganic acid salts (including hydrochloride, hydrobromide, hydroiodide, sulfate, nitrate, and phosphate); salts with amino acids (including arginine, aspartate, and glutamate); metal salts such as alkali metal salts (including sodium salts and potassium salts) and alkaline earth metal salts (including calcium salts and magnesium salts); ammonium salts; organic base salts (including trimethylamine salts, triethylamine salts, pyridine salts, picolinate, dicyclohexylamine salts, and N,N'-dibenzylethylenediamine salts); and other salts which a person skilled in the art can optionally select.

The present compounds of formula (1) and their intermediates can be isolated and purified in a manner known by a skilled person. It includes, for example, extraction, partition, reprecipitation, column chromatography (e.g. silica gel column chromatography, ion exchange column chromatography, and preparative liquid chromatography), and recrystallization.

The solvent for recrystallization used herein includes, for example, an alcohol solvent such as methanol, ethanol, and 2-propanol; an ether solvent such as diethyl ether; an ester solvent such as ethyl acetate; an aromatic hydrocarbon solvent such as benzene and toluene; a ketone solvent such as acetone; a halogenated solvent such as dichloromethane and chloroform; a hydrocarbon solvent such as hexane; an aprotic solvent such as dimethylformamide and acetonitrile; water; and a mixed solvent thereof. As other methods for purification, for example, methods described in Series of Experimental Chemistry, Vol. 1 or other volumes (Jikken Kagaku Kouza, edited by the Chemical Society of Japan, issued by MARUZEN) can be used. And, the structure of the present compounds can be easily determined by spectroscopic analytical method such as nuclear magnetic resonance method, infrared absorption method, and circular dichroism spectral analysis, and/or mass spectrometry, considering the structure of each starting compound.

In Lewy body disease such as Parkinson disease, abnormally aggregated α-synuclein is found in the patient's brain. Accordingly, the medicament of the present invention that suppresses or reduces the accumulation of α-synuclein aggregates is expected to exert an effect of improving the condition of these diseases.

In addition, it is believed that the aggregate exhibits neurotoxicity, induces neuronal vulnerability and neuronal cell death, and causes the onset and progression of pathology. Accordingly, the medicament of the present invention that suppresses neurotoxicity and neuronal cell death associated with α-synuclein aggregates is expected to exert an effect of improving the pathology of Lewy body disease such as Parkinson disease.

Neurotransmitter production is one of neuronal functions, and neurotransmitter reduction indicates neural vulnerability. For example, in dopaminergic neurons, the neural vulnerability is indicated by a decrease in the amount of tyrosine hydroxylase involved in dopamine metabolism.

Furthermore, abnormalities in electroencephalograms have been reported in Lewy body diseases such as Parkinson disease. Electroencephalographic activity is a manifestation of neurosynchronous activity. Accordingly, the medicament of the present invention that normalizes neuronal synchronous activity associated with α-synuclein aggregates is expected to exert an effect of improving the pathology on these diseases.

The neurospheroid used for measuring the amount of α-synuclein aggregate can be prepared, for example, by three-dimensional culture of neural stem cells prepared from human iPS cells having gene mutation related to synucleinopathy, or dopaminergic (DA) neural progenitor cells, under induction of neural differentiation. The amount of α-synuclein aggregate can be evaluated by measuring the amount of α-synuclein having high molecular weight by means of protein analysis using an α-synuclein antibody with neurospheroids prepared by three-dimensional culture.

The synchronous neural firing can be evaluated by means of imaging analysis using a fluorescent calcium probe with neurospheroids prepared by three-dimensional culture.

In addition, by using the process of measuring the amount of α-synuclein aggregate and the process of measuring the synchronous neural firing in the neurospheroid, Parkinson disease pathology can be reproduced and the drug evaluation about the action of suppressing or reducing the accumulation of α-synuclein aggregate in Parkinson disease pathology can be performed.

The induction of differentiation from geneticallymutated human iPS cells which are related to synucleinopathy to neural stem cell can be carried out, for example, by culturing PLA2G6 gene mutant cells established from healthy human-derived iPS cell line (Clone name: 201B7, obtained from iPS Cell Research and Application, Kyoto University) in StemFitAK03N medium (AJINOMOTO CO., INC., Basic03) at 37°C under 5% CO₂, and inducing the cultured product with PSC Neuronal Induction Medium (Thermo Fisher Scientific Inc., cat# A1647801).

As a culture medium for neural stem cells, for example, the following compositions can be used.

### <Culture medium composition for neural stem cells>

Neurobasal medium (Thermo Fisher Scientific Inc., 2113049)
Advanced DMEM/F-12 medium (Thermo Fisher Scientific Inc., 12634028)
Neural Induction Supplement (Thermo Fisher Scientific Inc., A1647801)

The induction of differentiation from neural stem cells to neurospheroid can be carried out, for example, by seeding neural stem cells (10000 cells/well) in 96-well round bottom plate (Thermo Fisher Scientific Inc., cat# 174929), culturing the cells in a culture medium at 37°C under 5% CO₂, and replacing half of the culture on day 2 and day 4 after the induction of differentiation.

As a culture medium for neurospheroid of neural stem cells, for example, the following compositions can be used.

### <Culture medium composition of neurospheroid>

BrainPhys Neuronal Medium (STEMCELL Technologies, cat# ST-05793)
NeuroCult SM1 Neuronal Supplement (STEMCELL Technologies, cat# 05711)
N2 Supplement-A (STEMCELL Technologies, cat# 07152)
20 ng/mL BDNF (PeproTech, Inc., cat# 450-02)
20 ng/mL GDNF (PeproTech, Inc., cat# 450-10)
1 mM dibutyryl cAMP (NACALAI TESQUE, INC., cat# 11540-74)
200 nM ascorbic acid (NACALAI TESQUE, INC., cat# 03420-52)

The induction of differentiation from geneticallymutated human iPS cells which are related to synucleinopathy to dopaminergic progenitor cells can be carried out, for example, by inducing dopaminergic progenitor cells from GBA1 gene homozygous mutant cells established from PLA2G6 gene mutant cells or healthy human-derived iPS cell line, with a dopaminergic neuron differentiation kit (Thermo Fisher Scientific Inc., cat# A3147701).

The induction of differentiation from dopaminergic progenitor cells to neurospheroid can be carried out, for example, by culturing cryopreserved dopaminergic progenitor cells at 37°C under 5% CO₂ with Floor Plate Cell expansion kit (Thermo Fisher Scientific Inc., cat# A3165801), seeding dopaminergic progenitor cells (10000 cells/well) in a 96-well round bottom plate (Thermo Fisher Scientific Inc., cat# 174929), culturing the cells in a culture medium at 37°C under 5% CO₂, and replacing half of the culture every 3 or 4 days after the induction of differentiation.

As a culture medium for dopamine neurospheroid from dopaminergic progenitor cells, for example, the following compositions can be used.

### <Culture medium composition of dopamine neurospheroid>

BrainPhys Neuronal Medium (STEMCELL Technologies, cat# ST-05793)
Dopaminergic Neuron Maturation Supplement (Thermo Fisher Scientific Inc., cat# A3147401)
20 ng/mL BDNF (PeproTech, Inc., cat# 450-02)
20 ng/mL GDNF (PeproTech, Inc., cat# 450-10)
1 mM dibutyryl cAMP (NACALAI TESQUE, INC., cat#11540-74)
200 nM ascorbic acid (NACALAI TESQUE, INC., cat#03420-52)

The measurement of the amount of α-synuclein aggregate in neurospheroid can be carried out, for example, by
taking out the differentiation-induced neurospheroid from the culture medium,
adding TBS solution (NACALAI TESQUE, INC., cat# 12748-31) containing 1% TritionX-100 (NACALAI TESQUE, INC., cat# 12967-32) thereto,
extracting the protein from the mixture with an ultrasonicator, and
subjecting the extracted protein to a protein analysis (Protein Simple, Inc., cat# SM-WO08) under non-reducing conditions with Simple Western system using an α-synuclein antibody (Thermo Fisher Scientific Inc., cat# AHB0261) to evaluate the quantitation on the waveform shown at a molecular weight of about 300 kD.

The measurement of the neural vulnerability in neurospheroid can be carried out, for example,
transferring the differentiation-induced dopamine neurospheroid into a TBS solution (NACALAI TESQUE, INC., cat# 12748-31) containing 1% TritionX-100 (NACALAI TESQUE, INC., cat# 12967-32),
extracting the protein from the mixture with an ultrasonicator, and
subjecting the extracted proteins to a protein analysis with Simple Western system (Protein Simple, Inc., cat# SM-WO04) using a tyrosine hydroxylase antibody (Millipore, cat# AB152) to evaluate the quantitation on the waveform shown at a molecular weight of about 60 kD.

The measurement of the neuronal cell death in neurospheroid can be carried out, for example, by
transferring the differentiation-induced dopamine neurospheroid into a TBS solution (NACALAI TESQUE, INC., cat# 12748-31) containing 1% TritionX-100 (NACALAI TESQUE, INC., cat# 12967-32),
extracting the protein from the mixture with an ultrasonicator, and
subjecting the extracted proteins to a protein analysis under reduced conditions with Simple Western system (Protein Simple, Inc., cat# SM-WO04) using cleaved caspase 3 antibody (Cell Signaling Technology, Inc., cat# 9664) to evaluate the quantitation on the waveform shown at a molecular weight of about 20 kD.

Abnormal nerve activity in neurospheroid can be measured by, for example, imaging analysis using a fluorescent calcium probe on three-dimensionally cultured neurospheroid to measure synchronous nerve firing.

The synchronous nerve firing in neurospheroid can be measured by, for example, imaging analysis using a measurement medium containing a fluorescent calcium probe (Molecular Devices, Product name: FLIPR Calcium 6 Assay Bulk Kit, cat# R8191).

The measurement medium used herein includes, for example, 20 mM Hepes (Thermo Fisher Scientific Inc., cat# 15630-080), and Hank's buffer solution (Thermo Fisher Scientific Inc., cat# 14065-056) containing 0.1 % bovine serum albumin (Sigma-Aldrich Co. LLC, cat# A9576).

The compound of the present invention is useful as a medicament for treating and/or preventing a central nervous system disease associated with abnormal protein aggregates in the brain.

The central nervous system disease associated with abnormal protein aggregates in the brain includes a central nervous system disease associated with tau, α-synuclein, TDP-43, or polyglutamine.

The central nervous system disease associated with tau includes Alzheimer disease, and frontotemporal lobar degeneration; the central nervous system disease associated with α-synuclein aggregates includes Parkinson disease, Lewy body dementia, multiple system atrophy, Gaucher disease, and infantile neuroaxonal dystrophy; the central nervous system disease associated with TDP-43 includes amyotrophic lateral sclerosis, and frontotemporal lobar degeneration; and the central nervous system disease associated with polyglutamine includes Huntington disease, and spinocerebellar ataxia.

The compound of the present invention is useful as a medicament for treating and/or preventing preferably Alzheimer disease, frontotemporal lobar degeneration, Parkinson disease, Lewy body dementia, multiple system atrophy, Gaucher disease, infantile neuroaxonal dystrophy, amyotrophic lateral sclerosis, Huntington disease, or spinocerebellar ataxia.

The compound of the present invention is useful as a medicament for treating and/or preventing more preferably a disease associated with α-synuclein aggregates.

The compound of the present invention is useful as a medicament for treating and/or preventing even more preferably Parkinson disease, Lewy body dementia, multiple system atrophy, Gaucher disease, or infantile neuroaxonal dystrophy.

In the present invention, the "prevention/preventing" means that the active ingredient of the present invention is administered to a healthy subject who does not suffer from the disease, for example, said purpose of the administration is for preventing the onset of the disease. The "treatment/treating" means that the active ingredient of the present invention is administered to a subject who is diagnosed with the disease by a physician (*i.e.*, a patient).

The compound of the present invention and the medicament comprising it can be orally or parenterally administered directly or as a suitable drug formulation. The formulation type includes, for example, a tablet, a capsule, a powder, a granule, a liquid, a suspension, an injection, a patch, a poultice, and the like, but it is not limited to them. The drug formulation is prepared by a common method using pharmaceutically acceptable additives.

As the additive, an excipient, a disintegrant, a binder, a fluidizer, a lubricant, a coating agent, a solubilizer, a solubilizing adjuvant, a thickener, a dispersant, a stabilizing agent, a sweetening agent, a flavor, and the like may be used, depending on purpose. The additive used herein includes, for example, lactose, mannitol, crystalline cellulose, lower-substituted hydroxypropylcellulose, corn starch, partially-pregelatinized starch, carmellose calcium, croscarmellose sodium, hydroxypropylcellulose, hydroxypropylmethylcellulose, polyvinyl alcohol, magnesium stearate, sodium stearyl fumarate, polyethylene glycol, propylene glycol, titanium oxide, talc, and the like.

The route of administration should be chosen to be the most effective route for the treatment, which includes oral administration and parenteral administration such as intravenous injection, swabbing, inhalation, and eyedrop. Preferred one is oral administration. The dosage form includes, for example, tablet and injection, and preferably tablet. The dose of the pharmaceutical composition and the frequency of administration thereof can depend on the administration route, and patient's disease, symptom, age, body weight, etc., thus it is impossible to define them flatly. In general, the present compound may be administered to an adult by about 0.0001 - about 5000 mg/day, preferably about 0.001 - about 1000 mg/day, more preferably about 0.1 - about 500 mg, particularly preferably about 1 - about 300 mg, which may be administered once a day or a few times a day, preferably once to three times a day.

The compound of the present invention and the medicament comprising it may be used together or combined with a different drug, in order to enhance the effect and/or reduce side effects. They may be used together with a medicament for treating central nervous system disease such as L-dopa, a dopamine agonist (for example, ropinirole hydrochloride, apomorphine hydrochloride hydrate, etc.), an MAO-B inhibitor (for example, selegiline hydrochloride, etc.), a catechol-O-methyltransferase (COMT) inhibitor (for example, entacapone, etc.), α-Syn antibody (for example, prasinezumab, etc.), and their pharmaceutically acceptable salts. Hereinafter, a medicament which may be used together with the compound of the present invention may be abbreviated as a "different drug (used together)".

The administration interval of the present compound, the medicament comprising it, and the different drug used together should not be limited. They may be simultaneously administered to a subject in need thereof, or may be administered with time interval. In addition, the present compound and the different drug may be mixed as a combination drug. The dose of the different drug used together may be defined suitably based on the clinically-used dose. The mixing ratio of the present compound and the different drug used together may be defined suitably depending on subject in need of the treatment, administration route, and subject's disease, symptom, combination, etc. When the subject in need of the treatment is human subject, the different drug used together may be used, for example, in a dose of 0.01 - 100 parts by weight per one part of the present compound. In order to suppress its side effect, a different drug used together such as an antiemetic drug, a sleep-inducing drug, an anticonvulsant drug may be used.

### EXAMPLES

The present invention is explained in more detail in the following by referring to Reference examples, Examples, and Tests; however, the technical scope of the present invention is not limited thereto. In the present specification, the term "Example" or "Reference example" sometimes means a compound itself, for example, "Example 1" indicates "a compound of Example 1", "Reference example 1" indicates "a compound of Reference example 1". The compound names used in Reference examples and Examples should not be always based on IUPAC nomenclature system.

The symbols used in NMR are defined as follows, s: singlet, d: doublet, dd: doublet of doublet, t: triplet, td: triplet of doublet, q: quartet, m: multiplet, br: broad, brs: broad singlet, brm: broad multiplet, and J: coupling constant.

Liquid Chromatography-Mass Spectrometry; Analytical conditions of LC/MS are shown below. Observed mass spectrometry value [MS(m/z)] is shown in MH⁺, and retention time is shown as Rt (minutes). In each measured result, A or B is added as the analytical condition used for the measurement.

### Analytical condition A

Detection Equipment:
MS detector: Waters ACQUITY SQ Detector
HPLC: Waters ACQUITY UPLC
Column: ACQUITY UPLC BEH C18 1.7 µm, 2.1 x 30 mm
Flow rate: 0.8mL/min
Column oven temperature: 40°C
Wave length: 254, 220 nm
Mobile phase:
A: 0.06 % formic acid/H₂O
B: 0.06 % formic acid/acetonitrile

Time program:

| Step | Time (min) | |
|---|---|---|
| 1 | 0.0-1.3 | A : B = 98 : 2 -> 4 : 96 |
| 2 | 1.3-1.5 | A : B = 4 : 96 -> 98 : 2 |

### Reference example 1

### 5-(Piperidin-4-yl)-2-(trifluoromethyl)pyridine

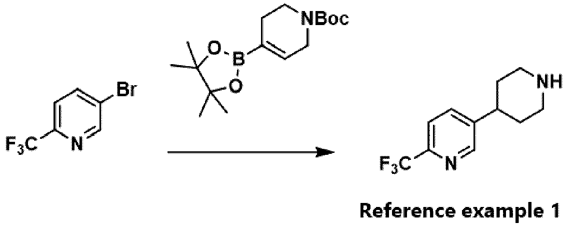

To a solution of 1-(tert-butoxycarbonyl)-1,2,3,6-tetrahydro-4-(4,4,5,5-tetramethyl-1,3,2-dioxaborolan-2-yl)pyridine (6.7 g) in cyclopentyl methyl ether/water (4/1) (100 mL) were added 5-bromo-2-(trifluoromethyl)pyridine (4.1 g), [1,1'-bis(diphenylphosphino)ferrocene]dichloropalladium(II) (0.66 g), and cesium carbonate (12 g), and the mixture was stirred at 100°C for 30 minutes. After cooling the reaction solution to room temperature, water was added thereto, and the mixture was extracted with ethyl acetate. The obtained organic layer was washed with brine and dried over magnesium sulfate. The solvent was removed *in vacuo* from the dried organic layer, and the residue was briefly purified by amino silica gel chromatography (elute solvent; hexane/ethyl acetate). The roughly-purified product was dissolved in methanol (50 mL), and 10% palladium carbon (55% wet) (0.30 g) was added thereto. The mixture was stirred under hydrogen atmosphere at room temperature for 3 hours. The reaction solution was filtered on Celite once and washed with methanol, and then the filtrate was concentrated under reduced pressure. The residue was dissolved in methanol (50 mL) again, and 10% palladium carbon (55% wet) (0.30 g) was added thereto. The mixture was stirred under hydrogen atmosphere at room temperature for 3 hours. The reaction solution was filtered on Celite and washed with methanol, and then the filtrate was concentrated under reduced pressure. The residue was dissolved in chloroform (10 mL), and trifluoroacetic acid (20 mL) was added thereto. The mixture was stirred at room temperature for one minute, and the solvent and trifluoroacetic acid were removed *in vacuo* from the mixture. The residue was purified by amino silica gel chromatography (elute solvent; hexane/ethyl acetate -> ethyl acetate/methanol) to give Reference example 1 (3.8 g).
LC/MS ([M+H]⁺/Rt(min)): 231.2/0.50 (Analytical condition A)

### Example 1

### (3-Methyloxetan-3-yl){4-[6-(trifluoromethyl)pyridin-3-yl]piperidin-1-yl}methanone

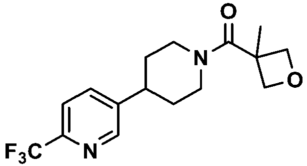

To a solution of Reference example 1 (30 mg) in chloroform (1.0 mL) were added 3-methyloxetane-3-carboxylic acid (18 mg), triethylamine (27 µL), and HATU (60 mg), and the mixture was stirred at room temperature for 30 minutes. And then, cesium carbonate (42 mg) was added thereto, and the mixture was stirred at room temperature for 30 minutes again. The reaction solution was purified by amino silica gel chromatography (elute solvent; hexane/ethyl acetate -> ethyl acetate/methanol) to give Example 1 (41 mg).
LC/MS ([M+H]⁺/Rt (min)): 329.2/0.75 (Analytical condition A) ¹H-NMR (400 MHz, DMSO-d6) δ: 8.72 (1H, d, J = 1.6 Hz), 8.02 (1H, dd, J = 2.0, 8.4 Hz), 7.84 (1H, d, J = 8.8 Hz), 4.83 (2H, dd, J = 6.4, 8.8 Hz), 4.55-4.52 (1H, m), 4.28 (2H, t, 6.8 Hz), 3.18-3.05 (2H, m), 3.01-2.93 (1H, m), 2.67 (1H, t, J = 12 Hz), 1.85-1.81 (2H, brs), 1.72-1.57 (5H, m).

The compound of Example 1 can be also prepared in a manner below.

To a solution of hydrochloride of Reference example 1 (100 mg) in ethyl acetate (1.0 mL) were added 3-methyloxetane-3-carboxylic acid (52 mg), triethylamine (0.17 mL), and a 50% solution of 2,4,6-tripropyl-1,3,5,2,4,6-trioxatriphosphorinane-2,4,6-trioxide in ethyl acetate (0.40 mL), and the mixture was stirred at room temperature for one hour. The reaction solution was purified by silica gel chromatography (elute solvent; ethyl acetate/methanol) to give Example 1 (93 mg).

### Example 2

### Preparation of (3-methyloxetan-3-yl){4-[6-(trifluoromethyl)pyridin-3-yl]piperidin-1-yl}methanone in crystalline form I

A solution of 5-(piperidin-4-yl)-2-(trifluoromethyl)pyridine hydrochloride (53 g), 3-methyloxetane-3-carboxylic acid (24 g), 1-ethyl-3-(3-dimethylaminopropyl)carbodiimide hydrochloride (40 g), and 1-hydroxybenzotriazole (2.7 g) in chloroform (286 mL) was cooled to 0°C. To the reaction solution was added triethylamine (56 mL) at 0°C, and the mixture was warmed to room temperature and stirred overnight. The reaction solution was washed with water, and the organic layer was washed with saturated aqueous sodium bicarbonate. The organic layer was dried over magnesium sulfate, and the solvent was removed off *in vacuo*. To the obtained crude product were added diethyl ether (100 mL) and hexane (20 mL), and the mixture was stirred at 0°C for 30 minutes to give a solid (49 g). The solid was purified by amino silica gel chromatography (elute solvent; hexane/ethyl acetate 90:10 - > 0:100) to obtain the desired fraction. The fraction was concentrated to dryness. Finally, the product was washed with diethyl ether (100 mL) to give Example 1 (37 g).

Example 1 (totally 136 g) which was similarly prepared by the above method was dissolved in ethyl acetate (200 mL), and the solution was stirred while heating at 80°C. To the solution was added hexane (300 mL), and the mixture was cooled to room temperature, and then stood at 0°C. The precipitated solid was collected on a filter, washed with hexane/ethyl acetate (3:2, 100 mL), and dried to isolate the compound of Example 1 in crystalline form I. The crystal was characterized below.

### Characterization of crystalline form

The crystalline form of the present compound was characterized using the procedures described below by various analytical techniques, including X-ray powder diffraction (XRPD), differential scanning calorimetry (DSC), thermogravimetry (TGA), and dynamic vapor sorption (DVS).

### Example 3:

### X-ray powder diffraction (XRPD) of the crystal in crystalline form I that was prepared in Example 2

XRPD analysis of (3-methyloxetan-3-yl){4-[6-(trifluoromethyl)pyridin-3-yl]piperidin-1-yl}methanone in crystalline form I was conducted under the following measurement conditions.

X-ray powder diffraction: XRPD analysis was conducted using a diffractometer (Bruker AXS D8 ADVANCE, Bruker, Billerica, Massachusetts, USA) with a copper X-ray tube (Cu Kα1, λ=1.5406 Å, Kα2, λ=1.5444 Å). The generator was operated at a voltage of 40 kV and a current of 40 mA. The sample for analysis was prepared by placing it at the center of a steel holder equipped with a zero-background plate. The slits used were a soller slit of 2.5°, a divergence slit of 0.2°, and an anti-scatter slit of 5.5 mm. The sample spinning rate was 0.25 revolutions per second. The scan was conducted over a diffraction angle (2θ) range of 2-40°, with a step size of 0.015° and a counting time of 48 seconds per step. Data analysis was performed using DIFFRAC.EVA (Bruker, Billerica, Massachusetts, USA).

XRPD analysis of (3-methyloxetan-3-yl){4-[6-(trifluoromethyl)pyridin-3-yl]piperidin-1-yl}methanone in crystalline form I was conducted under the measurement conditions described above. The XRPD pattern is shown in Table 1 and Figure 1.

**[Table 1]**

| Peak position (2Θ) | relative intensity | Peak position (2Θ) | relative intensity |
|---|---|---|---|
| 13.73 | 7.6% | 19.906 | 19.8% |
| 16.552 | 24.5% | 20.073 | 8.4% |
| 16.878 | 5.3% | 21.976 | 11.5% |
| 17.235 | 17.2% | 22.842 | 8.6% |
| 17.817 | 36.7% | 23.903 | 13.5% |
| 18.281 | 17.6% | 25.182 | 15.2% |
| 19.004 | 4.5% | 26.864 | 6.7% |
| 19.647 | 100% | 30.529 | 5.3% |

### Example 4:

### Differential scanning calorimetry (DSC) of the crystal in crystalline form I that was prepared in Example 2

Differential scanning calorimetry of (3-methyloxetan-3-yl){4-[6-(trifluoromethyl)pyridin-3-yl]piperidin-1-yl}methanone in crystalline form I was conducted under the following procedure.

Differential scanning calorimetry: Thermal properties were evaluated using a differential scanning calorimetry (DSC) instrument (DSC2500, TA Instruments, New Castle, DE, USA). Approximately 1 - 10 mg of the solid sample was placed in a T_ZERO sample pan (TA Instruments) and heated at a rate of 10°C/min under a nitrogen purge of 50 mL/min. Data analysis was performed using TRIOS (TA Instruments, New Castle, DE, USA).

The DSC curve of (3-methyloxetan-3-yl){4-[6-(trifluoromethyl)pyridin-3-yl]piperidin-1-yl}methanone in crystalline form I is shown in Figure 2. Crystalline form I has a melting point of 100°C or higher and exhibits high crystalline stability.

### Example 5:

### Thermogravimetry (TGA) of the crystal in crystalline form I that was prepared in Example 2

Thermogravimetry of (3-methyloxetan-3-yl){4-[6-(trifluoromethyl)pyridin-3-yl]piperidin-1-yl}methanone in crystalline form I was conducted under the following procedure.

Thermogravimetry: Thermogravimetry (TGA) was conducted using a TGA instrument (TGA 5500, TA Instruments, New Castle, DE, USA). Approximately 1 - 10 mg of the solid sample was placed in an open aluminum pan and heated at a rate of 10°C/min under a nitrogen purge of 25 mL/min. Data analysis was performed using TRIOS (TA Instruments, New Castle, DE, USA).

The TGA curve of (3-methyloxetan-3-yl){4-[6-(trifluoromethyl)pyridin-3-yl]piperidin-1-yl}methanone in crystalline form I is shown in Figure 3. Crystalline form I exhibits no weight loss up to the melting temperature and is therefore suitable for formulation development.

### Example 6:

### Dynamic vapor sorption (DVS) of the crystal in crystalline form I that was prepared in Example 2

Dynamic vapor sorption measurement of (3-methyloxetan-3-yl){4-[6-(trifluoromethyl)pyridin-3-yl]piperidin-1-yl}methanone in crystalline form I was conducted under the following procedure.

Dynamic vapor sorption: Hygroscopicity was evaluated at room temperature using a dynamic vapor sorption (DVS) instrument (IGAsorp, Hiden Isochema, Warrington, UK). Water sorption and desorption were investigated at 25°C over a relative humidity (RH) range of 0 - 90% as a function of relative humidity. The relative humidity in the chamber was increased in increments of 10% RH and maintained until equilibrium was reached between the solid sample and the atmosphere. At this point, the RH was increased by 10%, and the process was repeated until 90% RH was reached and equilibration was achieved. The moisture sorption was monitored during this period. For desorption, the relative humidity was reduced in a similar manner, and a complete sorption/desorption cycle was measured. The cycle was repeated as necessary. All experiments were operated in dm/dt mode (mass change over time) to determine the equilibration endpoint. Approximately 5 - 20 mg of the solid was used. Data analysis was performed using Hisorp (Hiden Isochema, Warrington, UK).

The DVS curve of (3-methyloxetan-3-yl){4-[6-(trifluoromethyl)pyridin-3-yl]piperidin-1-yl}methanone in crystalline form I is shown in Figure 4.

### Example 7:

### Preparation of (3-methyloxetan-3-yl){4-[6-(trifluoromethyl)pyridin-3-yl]piperidin-1-yl}methanone in crystalline form I (Alternative method 1)

5 mg of (3-methyloxetan-3-yl){4-[6-(trifluoromethyl)pyridin-3-yl]piperidin-1-yl}methanone obtained in Example 2 was dissolved in each solvent listed in Table 2 at 85°C. Thereafter, crystallization was carried out under three conditions - room temperature, refrigeration, and drying - to obtain (3-methyloxetan-3-yl){4-[6-(trifluoromethyl)pyridin-3-yl]piperidin-1-yl}methanone in crystalline form I.

**[Table 2]**

| Condition | Solvent | Solvent volume (µL) |
|---|---|---|
| 1 | water | 100 |
| 2 | methanol | 25 |
| 3 | ethanol | 25 |
| 4 | isopropanol | 25 |
| 5 | chloroform | 25 |
| 6 | acetonitrile | 25 |
| 7 | 1,2-dimethoxyethane | 25 |
| 8 | tetrahydrofuran | 25 |
| 9 | 1,4-dioxane | 25 |
| 10 | diisopropyl ether | 200 |
| 11 | cyclopropyl methyl ether | 50 |
| 12 | ethyl acetate | 25 |
| 13 | isopropyl acetate | 25 |
| 14 | isobutyl acetate | 25 |
| 15 | acetone | 25 |
| 16 | chlorobenzene | 25 |
| 17 | toluene | 25 |
| 18 | xylene | 25 |
| 19 | water/methanol mixture (1:1) | 25 |
| 20 | water/isopropanol mixture (1:1) | 25 |
| 21 | water/acetone mixture (1:1) | 50 |
| 22 | water/acetonitrile mixture (1:1) | 25 |
| 23 | water/tetrahydrofuran mixture (1:1) | 25 |
| 24 | water/1,4-dioxane mixture (1:1) | 25 |
| 25 | water/ethyl acetate mixture (1:1) | 25 |
| 26 | heptane/acetone mixture (1:1) | 25 |
| 27 | heptane/acetonitrile mixture (1:1) | 25 |
| 28 | heptane/tetrahydrofuran mixture (1:1) | 25 |
| 29 | heptane/toluene mixture (1:1) | 25 |

### Example 8:

### Preparation of (3-methyloxetan-3-yl){4-[6-(trifluoromethyl)pyridin-3-yl]piperidin-1-yl}methanone in crystalline form I (Alternative method 2)

5 mg of (3-methyloxetan-3-yl){4-[6-(trifluoromethyl)pyridin-3-yl]piperidin-1-yl}methanone obtained in Example 2 was dissolved in each solvent listed in Table 3 at 85°C. Thereafter, the solution was rapidly cooled and stored in a freezer (-20°C) to induce crystallization, thereby obtaining (3-methyloxetan-3-yl){4-[6-(trifluoromethyl)pyridin-3-yl]piperidin-1-yl}methanone in crystalline form I.

**[Table 3]**

| Condition | Solvent | Solvent volume (µL) |
|---|---|---|
| 1 | methanol | 25 |
| 2 | isopropanol | 25 |
| 3 | 1,2-dimethoxyethane | 25 |
| 4 | 1,4-dioxane | 25 |
| 5 | diisopropyl ether | 400 |
| 6 | cyclopropyl methyl ether | 50 |
| 7 | ethyl acetate | 25 |
| 8 | acetone | 25 |
| 9 | chlorobenzene | 25 |
| 10 | toluene | 25 |
| 11 | xylene | 25 |

### Example 9:

### Preparation of (3-methyloxetan-3-yl){4-[6-(trifluoromethyl)pyridin-3-yl]piperidin-1-yl}methanone in crystalline form I (Alternative method 3)

15 - 30 mg of (3-methyloxetan-3-yl){4-[6-(trifluoromethyl)pyridin-3-yl]piperidin-1-yl}methanone obtained in Example 2 was suspended in 50 - 500 µL of each solvent shown in Table 4, and the suspension was shaken for 14 days under two conditions, room temperature/50°C, thereby obtaining (3-methyloxetan-3-yl){4-[6-(trifluoromethyl)pyridin-3-yl]piperidin-1-yl}methanone in crystalline form I.

**[Table 4]**

| Condition | Solvent |
|---|---|
| 1 | water |
| 2 | ethanol |
| 3 | isopropanol |
| 4 | chloroform |
| 5 | acetonitrile |
| 6 | tetrahydrofuran |
| 7 | ethyl acetate |
| 8 | acetone |
| 9 | chlorobenzene |
| 10 | toluene |
| 11 | water/ethanol mixture (1:1) |
| 12 | water/isopropanol mixture (1:1) |
| 13 | water/tetrahydrofuran mixture (1:1) |
| 14 | water/acetone mixture (1:1) |
| 15 | water/acetonitrile mixture (1:1) |

### Test 1: Reproduction test of Parkinson disease pathology (accumulation of α-synuclein aggregate) with neurospheroid prepared by three-dimensional culture of human iPS cells with a mutation in PLA2G6 gene

PLA2G6 gene mutant cells established from healthy human-derived iPS cell line (Clone name: 201B7, obtained from iPS Cell Research and Application, Kyoto University) were cultured in StemFitAK03N medium (AJINOMOTO CO., INC., Basic03) at 37°C under 5% CO₂.

Neural stem cells were induced from iPS cells with PSC Neuronal Induction Medium (Thermo Fisher Scientific Inc., cat# A1647801) to prepare their cell stock.

The cryopreserved neural stem cells were cultured in culture medium at 37°C under 5% CO₂. The culture medium used herein for neural stem cells had the following composition.
Culture medium composition for neural stem cell
Neurobasal medium (Thermo Fisher Scientific Inc., 2113049) Advanced DMEM/F-12 medium (Thermo Fisher Scientific Inc., 12634028)
Neural Induction Supplement (Thermo Fisher Scientific Inc., A1647801)

The neural stem cells (10000 cells/well) were seeded in 96-well round bottom plate (Thermo Fisher Scientific Inc., cat# 174929), and cultured in a culture medium at 37°C under 5% CO₂. Half of the culture solution was replaced every 3 to 4 days. The culture medium used herein for neurospheroid had the following composition.
BrainPhys Neuronal Medium (STEMCELL Technologies, cat# ST-05793)
NeuroCult SM1 Neuronal Supplement (STEMCELL Technologies, cat# 05711)
N2 Supplement-A (STEMCELL Technologies, cat# 07152)
20 ng/mL BDNF (PeproTech, Inc., cat# 450-02)
20 ng/mL GDNF (PeproTech, Inc., cat# 450-10)
1 mM dibutyryl cAMP (NACALAI TESQUE, INC., cat# 11540-74)
200 nM ascorbic acid (NACALAI TESQUE, INC., cat# 03420-52)

The differentiated neurospheroid was taken out from the culture medium, TBS solution (NACALAI TESQUE, INC., cat# 12748-31) containing 1% TritionX-100 (NACALAI TESQUE, INC., cat# 12967-32) was added thereto, and the protein was extracted from the mixture with an ultrasonicator.

The extracted protein was subjected to a protein analysis (Protein Simple, Inc., cat# SM-WO08) under non-reducing conditions with Simple Western system using an α-synuclein antibody (Thermo Fisher Scientific Inc., cat# AHB0261) to measure the amount of the α-synuclein aggregate and evaluate the quantitation on the waveform shown at a molecular weight of about 300 kD.

The α-synuclein aggregate increased sharply from day 7 to day 9 of the culture. On day 9 of the culture, the amount of the α-synuclein aggregate in the neurospheroid derived from PLA2G6 mutant iPS cells was 5 times or more than that of the neurospheroid derived from healthy iPS cells. After day 9 of the culture, it showed a slow increasing trend. The result is shown in Fig. 5.

### Test 2: Evaluation of suppressing the accumulation of α-synuclein aggregate with neurospheroid prepared from human iPS cells with a mutation in PLA2G6 gene

### (1) Induction of differentiation from human iPS cell to neural cell

Neural stem cells were induced from PLA2G6 gene mutant cells with PSC Neuronal Induction Medium (Thermo Fisher Scientific Inc., cat# A1647801). Neurospheroid was prepared from the induced neural stem cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium was replaced on day 2 and day 4 after the induction of differentiation.

The test compound was diluted with the culture medium so as to be twice the final concentration, and an equal volume of the twice-concentrated solution was added to each well when half of the culture medium was replaced on day 4 after induction of differentiation.

### (2) Evaluation of the amount of α-synuclein aggregate

From the neurospheroid 9 days after the induction of differentiation, proteins were extracted with a TBS solution containing 1% TritionX-100, and the extracted proteins were subjected to a protein analysis with Simple Western system (Protein Simple, Inc., cat# SM-WO08) using an α-synuclein antibody (Thermo Fisher Scientific Inc., cat# AHB0261) to measure the amount of the α-synuclein aggregate.

The amount of the aggregate in the neurospheroid added with each test compound was evaluated. Assuming that the amount of the aggregate in the neurospheroid added with a DMSO solution was taken as 100%, the amount of the aggregate upon addition of the compound of Example 1 was 14% at 100 nM and 16% at 1000 nM.

### Test 3: Evaluation of reducing the accumulation of α-synuclein aggregate with neurospheroid prepared from human iPS cells with a mutation in PLA2G6 gene

### (1) Induction of differentiation from human iPS cell to neural cell

Neural stem cells were induced from PLA2G6 gene mutant cells with PSC Neuronal Induction Medium (Thermo Fisher Scientific Inc., cat# A1647801). Neurospheroid was prepared from the induced neural stem cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium was replaced every 3 or 4 days after the induction of differentiation.

The test compound was diluted with the culture medium so as to be twice the final concentration, and an equal volume of the twice-concentrated solution was added to each well when half of the culture medium was replaced on day 10 after induction of differentiation.

### (2) Evaluation of the amount of α-synuclein aggregate

From the neurospheroid 15 days after the induction of differentiation, proteins were extracted with a TBS solution containing 1% TritionX-100, and the extracted proteins were subjected to a protein analysis with Simple Western system (Protein Simple, Inc., cat# SM-WO08) using an α-synuclein antibody (Thermo Fisher Scientific Inc., cat# AHB0261) to measure the amount of the α-synuclein aggregate.

The amount of the aggregate in the neurospheroid added with each test compound was measured. Assuming that the amount of the aggregate in the neurospheroid added with a DMSO solution was taken as 100%, the amount of the aggregate upon addition of the compound of Example 1 was 20% at 100 nM and 30% at 1000 nM.

### Test 4: Reproduction test of Parkinson disease pathology (accumulation of α-synuclein aggregate) with dopamine neurospheroid prepared from human iPS cells with a mutation inPLA2G6 gene

Dopaminergic progenitor cells were induced from PLA2G6 gene mutant cells with dopaminergic neuron differentiation kit (Thermo Fisher Scientific Inc., cat# A3147701) to prepare their cell stock.

The cryopreserved dopaminergic progenitor cells were cultured at 37°C under 5% CO₂ with Floor Plate Cell expansion kit (Thermo Fisher Scientific Inc., cat# A3165801).

First, the dopaminergic progenitor cells (10000 cells/well) were seeded in a 96-well round-bottom plate (Thermo Fisher Scientific Inc., cat# 174929), and cultured in a culture medium at 37°C under 5% CO₂. Half of the culture medium was replaced every 3 or 4 days after the induction of differentiation. The culture medium used herein for dopamine neurospheroid had the following composition.
BrainPhys Neuronal Medium (STEMCELL Technologies, cat# ST-05793)
Dopaminergic Neuron Maturation Supplement (Thermo Fisher Scientific Inc., cat# A3147401)
20 ng/mL BDNF (PeproTech, Inc., cat# 450-02)
20 ng/mL GDNF (PeproTech, Inc., cat# 450-10)
1 mM dibutyryl cAMP (NACALAI TESQUE, INC., cat# 11540-74)
200 nM ascorbic acid (NACALAI TESQUE, INC., cat# 03420-52)

The differentiated dopamine neurospheroid was taken out from the culture medium, TBS solution (NACALAI TESQUE, INC., cat# 12748-31) containing 1% TritionX-100 (NACALAI TESQUE, INC., cat# 12967-32) was added thereto, and the protein was extracted from the mixture with an ultrasonicator.

The extracted protein was subjected to a protein analysis (Protein Simple, Inc., cat# SM-WO08) under non-reducing conditions with Simple Western system using an α-synuclein antibody (Thermo Fisher Scientific Inc., cat# AHB0261) to measure the amount of the α-synuclein aggregate and evaluate the quantitation on the waveform shown at a molecular weight of about 300 kD.

The α-synuclein aggregate increased sharply from day 10 to day 21 of the culture. On day 21 of the culture, the amount of the α-synuclein aggregate in the dopamine neurospheroid derived from PLA2G6 mutant iPS cells was 5 times or more than that of the dopamine neurospheroid derived from healthy iPS cells. After day 21 of the culture, it showed a slow increasing trend. The result is shown in Fig. 6.

### Test 5: Evaluation of suppressing the accumulation of α-synuclein aggregate with dopamine neurospheroid prepared from human iPS cells with a mutation in PLA2G6 gene

### (1) Induction of differentiation from human iPS cell to neural cell

Dopaminergic progenitor cells were induced from PLA2G6 gene mutant cells with dopaminergic neuron differentiation kit (ThermoFisher Inc., cat# A3147701). Dopamine neurospheroid was prepared from the induced dopaminergic progenitor cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium was replaced every 3 or 4 days after the induction of differentiation.

The test compound was diluted with the culture medium so as to be twice the final concentration, and an equal volume of the twice-concentrated solution was added to each well when half of the culture medium was replaced on day 21 after induction of differentiation.

### (2) Evaluation of the amount of α-synuclein aggregate

From the dopamine neurospheroid 26 days after the induction of differentiation, proteins were extracted with a TBS solution containing 1% TritionX-100, and the extracted proteins were subjected to a protein analysis with Simple Western system (Protein Simple, Inc., cat# SM-WO08) using an α-synuclein antibody (ThermoFisher Inc., cat# AHB0261) to measure the amount of the α-synuclein aggregate.

The amount of the aggregate in the neurospheroid added with each test compound was measured. Assuming that the amount of the aggregate in the neurospheroid added with a DMSO solution was taken as 100%, the amount of the aggregate upon addition of the compound of Example 1 was 17% at 100 nM and 21% at 1000 nM.

### Test 6: Reproduction method of neural vulnerability with dopamine neurospheroid prepared from human iPS cells with a mutation in PLA2G6 gene

### (1) Induction of differentiation from human iPS cell to neural cell

Dopaminergic progenitor cells were induced from PLA2G6 gene mutant cells with dopaminergic neuron differentiation kit (ThermoFisher Inc., cat# A3147701). Dopamine neurospheroid was prepared from the induced dopaminergic progenitor cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium was replaced every 3 or 4 days after the induction of differentiation.

### (2) Evaluation of the amount of tyrosine hydroxylase

From the dopamine neurospheroid 26 days after the induction of differentiation, proteins were extracted with a TBS solution containing 1% TritionX-100, and the extracted proteins were subjected to a protein analysis with Simple Western system (Protein Simple, Inc., cat# SM-WO08) using a tyrosine hydroxylase antibody (Millipore, cat# AB152) to measure the amount of the tyrosine hydroxylase*.* The result is shown in Fig. 7.

### Test 7: Evaluation of improving neural vulnerability with dopamine neurospheroid prepared from human iPS cells with a mutation in PLA2G6 gene

### (1) Induction of differentiation from human iPS cell to neural cell

Dopaminergic progenitor cells are induced from PLA2G6 gene mutant cells with dopaminergic neuron differentiation kit (ThermoFisher Inc., cat# A3147701). Dopamine neurospheroid is prepared from the induced dopaminergic progenitor cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium is replaced every 3 or 4 days.

The test compound is diluted with the culture medium so as to be twice the final concentration, and an equal volume of the twice-concentrated solution is added to each well when half of the culture medium is replaced on day 21 after induction of differentiation.

### (2) Evaluation of the amount of tyrosine hydroxylase

From the dopamine neurospheroid 26 days after the induction of differentiation, proteins are extracted with a TBS solution containing 1% TritionX-100, and the extracted proteins are subjected to a protein analysis with Simple Western system (Protein Simple, Inc., cat# SM-WO08) using a tyrosine hydroxylase antibody (Millipore, cat# AB152) to measure the amount of the tyrosine hydroxylase*.*

### Test 8: Reproduction method of neuronal cell death with neurospheroid prepared from human iPS cells with a mutation in PLA2G6 gene

### (1) Induction of differentiation from human iPS cell to neural cell

Dopaminergic progenitor cells were induced from PLA2G6 gene mutant cells with dopaminergic neuron differentiation kit (ThermoFisher Inc., cat# A3147701). Dopamine neurospheroid was prepared from the induced dopaminergic progenitor cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium was replaced every 3 or 4 days.

### (2) Evaluation of neuronal cell death

10 µM dopamine was added to the culture medium 35 days after the induction of differentiation. From the dopamine neurospheroid 40 days after the induction of differentiation, proteins were extracted with a TBS solution containing 1% TritionX-100, and the extracted proteins were subjected to a protein analysis with Simple Western system (Protein Simple, Inc., cat# SM-WO08) using cleaved caspase 3 antibody (Cell Signaling Technology, Inc., cat# 9664) to measure neuronal cell death. The result is shown in Fig. 8.

### Test 9: Evaluation of suppressing neuronal cell death with neurospheroid prepared from human iPS cells with a mutation in PLA2G6 gene

### (1) Induction of differentiation from human iPS cell to neural cell

Dopaminergic progenitor cells are induced from PLA2G6 gene mutant cells with dopaminergic neuron differentiation kit (ThermoFisher Inc., cat# A3147701). Dopamine neurospheroid is prepared from the induced dopaminergic progenitor cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium is replaced every 3 or 4 days.

The test compound is diluted with the culture medium so as to be twice the final concentration, and an equal volume of the twice-concentrated solution with 10 µM dopamine is added to each well when half of the culture medium is replaced on day 35 after induction of differentiation.

### (2) Evaluation of neuronal cell death

From the dopamine neurospheroid 40 days after the induction of differentiation, proteins are extracted with a TBS solution containing 1% TritionX-100, and the extracted proteins are subjected to a protein analysis with Simple Western system (Protein Simple, Inc., cat# SM-WO08) using a cleaved caspase 3 antibody (Cell Signaling Technology, Inc., cat# 9664) to measure the amount of the neuronal cell death*.*

### Test 10: Reproduction method of Parkinson disease pathology (accumulation of α-synuclein aggregate) with neurospheroid prepared by three-dimensional culture of human iPS cells with a mutation in GBA1 gene

GBA1 gene homozygous mutant cells established from healthy human-derived iPS cell line was cultured in StemFitAK03N medium (AJINOMOTO CO., INC., Basic03) at 37°C under 5% CO₂.

Dopaminergic progenitor cells were induced from GBA1 homozygous mutant iPS cells with dopaminergic neuron differentiation kit (ThermoFisher Inc., cat# A3147701) to prepare their cell stock.

The cryopreserved dopaminergic progenitor cells were cultured in a culture medium at 37°C under 5% CO₂ with Floor Plate Cell expansion kit (Thermo Fisher Scientific Inc., cat# A3165801).

The dopaminergic progenitor cells (10000 cells/well) were seeded in 96-well round bottom plate (Thermo Fisher Scientific Inc., cat# 174929), and cultured in a culture medium at 37°C under 5% CO₂. Half of the culture solution was replaced every 3 to 4 days after the induction of differentiation. The culture medium used herein for dopamine neurospheroid had the following composition.
BrainPhys Neuronal Medium (STEMCELL Technologies, cat# ST-05793)
Dopaminergic Neuron Maturation Supplement (Thermo Fisher Scientific Inc., cat# A3147401)
20 ng/mL BDNF (PeproTech, Inc., cat# 450-02)
20 ng/mL GDNF (PeproTech, Inc., cat# 450-10)
1 mM dibutyryl cAMP (NACALAI TESQUE, INC., cat# 11540-74)
200 nM ascorbic acid (NACALAI TESQUE, INC., cat# 03420-52)

The differentiation-induced dopamine neurospheroid was taken out from the culture medium, TBS solution (NACALAI TESQUE, INC., cat# 12748-31) containing 1% TritionX-100 (NACALAI TESQUE, INC., cat# 12967-32) was added thereto, and the protein was extracted from the mixture with an ultrasonicator.

The extracted protein was subjected to a protein analysis under non-reducing conditions with Simple Western system (Protein Simple, Inc., cat# SM-WO08) using an α-synuclein antibody (Thermo Fisher Scientific Inc., cat# AHB0261) to measure the amount of the α-synuclein aggregate and evaluate the quantitation on the waveform shown at a molecular weight of about 300 kD.

The α-synuclein aggregate increased sharply from day 21 to day 40 of the culture. On day 40 of the culture, the amount of the aggregate reached saturation, after which no change in the amount was observed. The result is shown in Fig. 9.

### Test 11: Evaluation of reducing the accumulation of α-synuclein aggregate with dopamine neurospheroid prepared from human iPS cells with a mutation in GBA1 gene

### (1) Induction of differentiation from human iPS cell to neural cell

Dopaminergic progenitor cells were induced from GBA1 gene mutant cells with dopaminergic neuron differentiation kit (ThermoFisher Inc., cat# A3147701). Dopamine neurospheroid was prepared from the induced dopaminergic progenitor cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium was replaced every 3 or 4 days.

The test compound was diluted with the culture medium so as to be twice the final concentration, and an equal volume of the twice-concentrated solution was added to each well when half of the culture medium was replaced on day 40 after induction of differentiation.

### (2) Evaluation of the amount of α-synuclein aggregate

From the dopamine neurospheroid 44 days after the induction of differentiation, proteins were extracted with a TBS solution containing 1% TritionX-100, and the extracted proteins were subjected to a protein analysis with Simple Western system (Protein Simple, Inc., cat# SM-WO08) using an α-synuclein antibody (ThermoFisher Inc., cat# AHB0261) to measure the amount of the α-synuclein aggregate.

The amount of the aggregate in the neurospheroid added with each test compound was measured. Assuming that the amount of the aggregate in the neurospheroid added with a DMSO solution was taken as 100%, the amount of the aggregate upon addition of the compound of Example 1 was 23% at 100 nM and 14% at 1000 nM.

### Test 12: Check of synchronous firing abnormality with neurospheroid prepared by three-dimensional culture of human iPS cells with a mutation in GBA1 gene

GBA1 gene homozygous mutant cells established from healthy human-derived iPS cell line is cultured in StemFitAK03N medium (AJINOMOTO CO., INC., Basic03) at 37°C under 5% CO₂.

Dopaminergic progenitor cells are induced from GBA1 homozygous mutant iPS cells with dopaminergic neuron differentiation kit (Thermo Fisher Scientific Inc., cat# A3147701) to prepare their cell stock.

The cryopreserved dopaminergic progenitor cells are cultured at 37°C under 5% CO₂ with Floor Plate Cell expansion kit (Thermo Fisher Scientific Inc., cat# A3165801).

The dopaminergic progenitor cells (10000 cells/well) are seeded in 96-well round bottom plate (Thermo Fisher Scientific Inc., cat# 174929), and cultured in a culture medium at 37°C under 5% CO₂. Half of the culture solution is replaced every 3 to 4 days after the induction of differentiation. The culture medium used herein for dopamine neurospheroid has the following composition.
BrainPhys Neuronal Medium (STEMCELL Technologies, cat# ST-05793)
Dopaminergic Neuron Maturation Supplement (Thermo Fisher Scientific Inc., cat#A3147401)
20 ng/mL BDNF (PeproTech, Inc., cat# 450-02)
20 ng/mL GDNF (PeproTech, Inc., cat# 450-10)
1 mM dibutyryl cAMP (NACALAI TESQUE, INC., cat# 11540-74)
200 nM ascorbic acid (NACALAI TESQUE, INC., cat# 03420-52)

On day 40 or later after the induction of differentiation, half of the culture medium is removed, a measurement medium containing fluorescent calcium probe (Molecular Devices, Product name: FLIPR Calcium 6 Assay Bulk Kit, cat#R8191) is added equal to the remaining medium, and the mixture is allowed to stand for 30 minutes and then measured. The measurement medium used herein is 20 mM Hepes (Thermo Fisher Scientific Inc., cat# 15630-080), and Hank's buffer solution (Thermo Fisher Scientific Inc., cat# 14065-056) containing 0.1 % bovine serum albumin (Sigma-Aldrich Co. LLC, cat# A9576). Shooting takes one frame per second.

### Test 13: Evaluation of improving synchronous firing abnormality with dopamine neurospheroid prepared by human iPS cells with a mutation in GBA1 gene

### (1) Induction of differentiation from human iPS cell to neural cell

Dopaminergic progenitor cells are induced from GBA1 gene mutant cells with dopaminergic neuron differentiation kit (ThermoFisher Inc., cat# A3147701). Dopamine neurospheroid is prepared from the induced dopaminergic progenitor cells by three-dimensional culture system, and preserved with BrainPhys Neuronal Medium (STEMCELL Technologies, cat# ST-05793) comprising NeuroCult SM1 Neuronal Supplement, N2 Supplement-A, 20 ng/mL BDNF, 20 ng/mL GDNF, 1 mM dibutyryl cAMP, and 200 nM ascorbic acid. Half of the culture medium is replaced every 3 or 4 days.

The test compound is diluted with the culture medium so as to be twice the final concentration, and an equal volume of the twice-concentrated solution is added to each well when half of the culture medium is replaced on day 40 after induction of differentiation.

### (2) Evaluation of synchronous firing

On day 44 after the induction of differentiation, half of the culture medium of dopamine neurospheroid is removed, a measurement medium containing fluorescent calcium probe (Molecular Devices, Product name: FLIPR Calcium 6 Assay Bulk Kit, cat# R8191) is added equal to the remaining medium, and the mixture is stood for 30 minutes and then measured. The measurement medium used herein is 20 mM Hepes (Thermo Fisher Scientific Inc., cat# 15630-080), and Hank's buffer solution (Thermo Fisher Scientific Inc., cat# 14065-056) containing 0.1 % bovine serum albumin (Sigma-Aldrich Co. LLC, cat# A9576). Shooting takes one frame per second.

### INDUSTRIAL APPLICABILITY

The present compound and a crystal thereof have an action of suppressing or reducing the accumulation of α-synuclein aggregates, and thereby the present compound is useful as a medicament for treating or preventing central nervous system disease (for example, Parkinson disease, Lewy body dementia, multiple system atrophy, Gaucher disease, infantile neuroaxonal dystrophy, etc.), which is characterized by an action of suppressing or reducing the accumulation of abnormal protein aggregates in the brain. In addition, a crystal of (3-methyloxetan-3-yl){4-[6-(trifluoromethyl)pyridin-3-yl]piperidin-1-yl}methanone in crystalline form I has a high melting point and excellent thermal stability and hygroscopic stability, thereby making it useful as a pharmaceutical product.

## Claims

1. A crystal of (3-methyloxetan-3-yl){4-[6-(trifluoromethyl)pyridin-3-yl]piperidin-1-yl}methanone in crystalline form I, which is **characterized by** X-ray powder diffraction pattern having diffraction angle (2θ°) peaks of 17.8° ± 0.2° and 19.6° ± 0.2°.

2. A crystal of (3-methyloxetan-3-yl){4-[6-(trifluoromethyl)pyridin-3-yl]piperidin-1-yl}methanone in crystalline form I, which is **characterized by** X-ray powder diffraction pattern having four or more diffraction angle (2θ°) peaks selected from 16.6° ± 0.2°, 17.2° ± 0.2°, 17.8° ± 0.2°, 18.3° ± 0.2°, 19.6° ± 0.2°, 19.9° ± 0.2°, 22.0° ± 0.2°, 22.8° ± 0.2°, 23.9° ± 0.2°, and 25.2° ± 0.2°.

3. A medicament comprising the crystal of (3-methyloxetan-3-yl){4-[6-(trifluoromethyl)pyridin-3-yl]piperidin-1-yl}methanone in crystalline form I according to claim 1 or 2 as an active ingredient.

4. A medicament for treating or preventing central nervous system diseases associated with abnormal protein aggregates in the brain, comprising the crystal of (3-methyloxetan-3-yl){4-[6-(trifluoromethyl)pyridin-3-yl]piperidin-1-yl}methanone in crystalline form I according to claim 1 or 2 as an active ingredient.

5. The medicament of claim 4, wherein the central nervous system diseases associated with abnormal protein aggregates in the brain are central nervous system diseases related to tau, α-synuclein, TDP-43, or polyglutamine.

6. The medicament of claim 4, wherein the central nervous system diseases associated with abnormal protein aggregates in the brain are Alzheimer disease, frontotemporal lobar degeneration, Parkinson disease, Lewy body dementia, multiple system atrophy, Gaucher disease, infantile neuroaxonal dystrophy, amyotrophic lateral sclerosis, Huntington disease, or spinocerebellar ataxia.

7. The medicament of claim 4, wherein the central nervous system diseases associated with abnormal protein aggregates in the brain are central nervous system diseases related to α-synuclein.

8. The medicament of claim 4, wherein the central nervous system diseases associated with abnormal protein aggregates in the brain are Parkinson disease, Lewy body dementia, multiple system atrophy, Gaucher disease, or infantile neuroaxonal dystrophy.

9. A method for treating and/or preventing central nervous system diseases associated with abnormal protein aggregates in the brain, comprising administering a therapeutically effective amount of the crystal of (3-methyloxetan-3-yl){4-[6-(trifluoromethyl)pyridin-3-yl]piperidin-1-yl}methanone in crystalline form I according to claim 1 or 2 to a patient in need thereof.

10. Use of the crystal of (3-methyloxetan-3-yl){4-[6-(trifluoromethyl)pyridin-3-yl]piperidin-1-yl}methanone in crystalline form I according to claim 1 or 2, in the manufacture of a medicament for treating and/or preventing central nervous system diseases associated with abnormal protein aggregates in the brain.

11. The crystal of (3-methyloxetan-3-yl){4-[6-(trifluoromethyl)pyridin-3-yl]piperidin-1-yl}methanone in crystalline form I according to claim 1 or 2 for use in treating and/or preventing central nervous system diseases associated with abnormal protein aggregates in the brain.

12. A medicament for treating or preventing central nervous system diseases associated with abnormal protein aggregates in the brain, comprising a combination of the crystal of (3-methyloxetan-3-yl){4-[6-(trifluoromethyl)pyridin-3-yl]piperidin-1-yl}methanone in crystalline form I according to claim 1 or 2, and at least one drug selected from the group consisting of L-dopa, dopamine agonist, MAO-B inhibitor, catechol-O-methyltransferase (COMT) inhibitor, αSyn antibody, and pharmaceutically acceptable salts thereof.

13. A medicament for treating or preventing central nervous system diseases associated with abnormal protein aggregates in the brain, comprising the crystal of (3-methyloxetan-3-yl){4-[6-(trifluoromethyl)pyridin-3-yl]piperidin-1-yl}methanone in crystalline form I according to claim 1 or 2, which is used in combination with at least one drug selected from the group consisting of L-dopa, dopamine agonist, MAO-B inhibitor, catechol-O-methyltransferase (COMT) inhibitor, αSyn antibody, and pharmaceutically acceptable salts thereof.
